# EUROPEAN PATENT APPLICATION

(11) **EP 3 547 054 A1**
(43) Date of publication of application: **02.10.2019**
(21) Application number: 17874950.3
(22) Date of filing: 22.11.2017
(51) Int. Cl.: G05B 19/418, G06Q 10/06, G06Q 50/04

(54) **PRODUCT MANUFACTURING METHOD AND PRODUCT MANUFACTURING DEVICE**

(30) Priority: 25.11.2016 JP 2016229251; 08.02.2017 JP 2017021494; 14.11.2017 JP 2017219350
(71) Applicant: Kao Corporation, Chuo-Ku Tokyo 103-8210 (JP)
(72) Inventor: TAIRA, Naohiro, Haga-gun Tochigi 321-3497 (JP); KOKUBO, Makoto, Haga-gun Tochigi 321-3497 (JP); UENO, Kota, Haga-gun Tochigi 321-3497 (JP)
(74) Representative: Vossius & Partner Patentanwälte Rechtsanwälte mbB
(86) International application number: PCT/JP2017/041989
(87) International publication number: WO 2018/097171

(57) **Abstract**

A method of producing a product 90 through a plurality of production steps, containing: a step of acquiring equipment data D3 of production equipment 40 for producing the product; a step of acquiring product data D1 from the product 90; a step of storing the equipment data D3 and the product data D1 in a data collection unit 70; a step of associating the equipment data D3 with the product data D1; a step of judging abnormality of the product data D1; a step responsive to abnormality occurring in the product 90 for identifying one or both of equipment abnormality data D3n and product abnormality data D1n associated with the product judged to be abnormal; and the step of identifying the production step causing the product abnormality by the step of identifying the abnormality data.

## Description

### FIELD OF THE INVENTION

The present invention relates to a method of producing a product to be produced, such as an absorbent article and a heating element, through a plurality of production steps; and a production apparatus therefor.

### BACKGROUND OF THE INVENTION

When a fault occurs in a product produced in a production line (when a defective product is found in a quality inspection step of the production process, or when complaints are received from consumers, or the like), cause thereof must be promptly investigated. Moreover, prompt investigation of cause is required not only in a case where a fault occurs in a produced product but also when a machine problem occurs in the production line.

Patent Literature 1 describes a quality information system in a production process, in which components are sequentially added to produce a composite product during production of a product. The components are formed of a continuous web material or a discontinuous web material. The quality information system is configured of an inspection system and a quality data subsystem. The inspection system automatically inspects quality of a sample set of the composite product to be produced to provide quality parameters in association with an inspected state. The quality data subsystem acquires and stores a plurality of quality parameters.

Patent Literature 2 describes a design method for redesigning product or process parameters using correlation data of product bar codes, off-line process control measured values, in-line sensor measured values and questionnaire (voice or video) results. Moreover, Patent Literature 2 discloses a quality tracking system in which quality information is generated from a database storing product identifier (serial number or bar code) information, production data, a recording module of feedback data from consumers, and correlations among respective products.

Further, Patent Literature 3 discloses a production method for an absorbent article (diaper). Inspection objects in the production method are a base material and a rear flap. The inspection is performed by a first sensor, a second sensor and a controller each connected to a communication network. Then, inspection parameters, process parameters and consumer feedback parameters are correlated with each other. Based on this correlation, a process parameter (applied pressure) is adjusted based on at least one of the inspection parameters or the consumer feedback parameters.

In recent years, communication networks have advanced in speed and capacity, and in combination with improved performance of inspection instruments, this has led to massive amounts of data being generated with respect to production line equipment and products.

### CITATION LIST

### PATENT LITERATURES

Patent Literature 1: WO 2004/015509
Patent Literature 2: WO 2015/034713
Patent Literature 3: WO 2015/034891

### SUMMARY OF THE INVENTION

The present invention provides a method of producing a product through a plurality of production steps, containing:
a step of acquiring equipment data of production equipment for producing the product;
a step of acquiring product data from the product;
a step of storing the equipment data and the product data in a data collection unit;
a step of associating the equipment data with the product data;
a step of judging abnormality of the product data;
a step responsive to abnormality occurring in the product for identifying one or both of equipment abnormality data and product abnormality data associated with the product judged to be abnormal; and
a step of further identifying the production step causing the product abnormality by the step of identifying the abnormality data.

The present invention provides an apparatus for producing a product, by which the product is produced correspondingly to a plurality of production steps, containing:
a plurality of pieces of production equipment by which the product is produced;
a plurality of inspection devices by which the product is inspected;
a sensor for detecting a production pattern possessed by the production equipment; and
a data collection unit in which product data obtained by the inspection devices and production pattern data detected by the sensor are stored in association with each other,
wherein the data collection unit has:
an auxiliary central processing unit in which the product data is processed,
a main central processing unit in which mutually associated equipment data indicating equipment states of the production equipment, auxiliary unit collection data processed in the auxiliary central processing unit and the production pattern data are processed, and
a database server in which main unit collection data processed in the main central processing unit are stored.

Other and further objects, features and advantages of the invention will appear more fully from the following description, appropriately referring to the accompanying drawings.

### BRIEF DESCRIPTION OF DRAWINGS

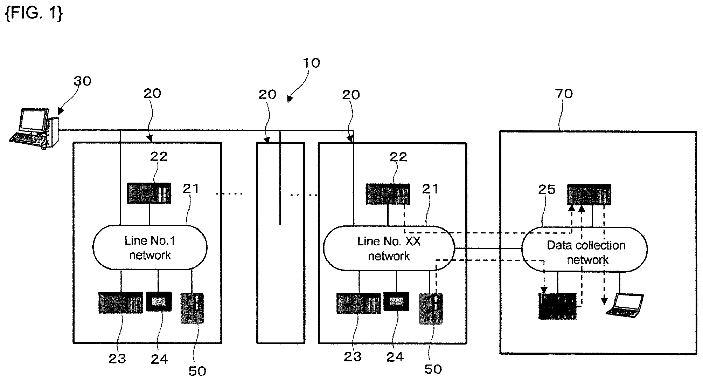

{FIG. 1}
   FIG. 1 is a schematic configuration diagram showing one preferable example of an apparatus for producing a product according to the present invention.
{FIG. 2}
   FIG. 2 is a schematic configuration diagram showing one preferable example of production equipment of the production apparatus for the same.
{FIG. 3}
   FIG. 3 is a schematic configuration diagram showing one preferable example of a production line and a data collection unit of the apparatus for producing a product shown in FIG. 1.
{FIG. 4}
   FIG. 4 is a block diagram showing one example of a detail of a data collection unit.
{FIG. 5}
   FIG. 5 is a schematic configuration diagram showing one example of association of data.
{FIG. 6}
   FIG. 6 is diagram showing one specific example of collection data indicating association of data.
{FIG. 7}
   FIG. 7 is a diagram showing one example of storage start and storage stop of collection data by a trigger.
{FIG. 8}
   FIG. 8 is a graph showing one example of a relationship between a stock diameter and time before and after splicing, a relationship between splicing and time and a relationship between an area value and time, in which respective time axes are aligned.
{FIG. 9}
   FIG. 9 is a process block diagram showing one example in which a method of producing a product is applied to a method of producing an absorbent article.
{FIG. 10}
   FIG. 10 is a configuration diagram showing one example of a fiber-stacking device as an absorbent body forming unit.
{FIG. 11}
   FIG. 11 is a cross-sectional view in a front-back direction of an absorbent body of a normal product.
{FIG. 12}
   FIG. 12 is a cross-sectional view in a front-back direction of an absorbent body of an abnormal product.
{FIG. 13}
   FIG. 13 is an image diagram showing one example of an image obtained by picking up a surface of an absorbent body of an abnormal product.
{FIG. 14}
   FIG. 14 is a plan view showing one example of a way of determining a gray value of an absorbent body.
{FIG. 15}
   FIG. 15 is a graph showing a relationship between a missing area value and a fiber-stacking suction frequency.
{FIG. 16}
   FIG. 16 is a schematic configuration diagram showing one example of a pressing unit of an absorbent body.
{FIG. 17}
   FIG. 17 is a longitudinal cross-sectional view of an absorbent body of a normal product.
{FIG. 18}
   FIG. 18 is a longitudinal cross-sectional view of an absorbent body of an abnormal product.
{FIG. 19}
   FIG. 19 is a relationship diagram between a height of an absorbent body and a measuring position, showing measurement results by a displacement sensor.
{FIG. 20}
   FIG. 20 is a schematic configuration diagram showing one example of an embossing unit.
{FIG. 21}
   FIG. 21 is a top view of an absorbent body of an abnormal product.
{FIG. 22}
   FIG. 22 is a top view of an absorbent body of a normal product.
{FIG. 23}
   FIG. 23 is a graph showing a relationship between an embossing indentation gray value and an embossing clearance.
{FIG. 24}
   FIG. 24 is a plan view showing one example of an image of an absorbent body having embossing.
{FIG. 25}
   FIG. 25 is a graph showing a relationship between an embossing indentation gray value and a missing area value (fiber-stacking suction frequency).
{FIG. 26}
   FIG. 26 is a configuration diagram showing one example of an apparatus for producing a heating element.
{FIG. 27}
   FIG. 27 is a relationship diagram between a height and a position, showing measurement results of a surface shape of a coating material layer.
{FIG. 28}
   FIG. 28 is a graph showing a relationship between heating element position accuracy (standard deviation) and a coating material cross-sectional area (the number of revolutions of a pump).

### DESCRIPTION OF EMBODIMENTS

The present invention relates to a method of producing a product and a production apparatus therefor, in which improvement in productivity is realized by utilizing data generated by production equipment. That is, the present invention relates to an art in which traceability of a defective product can be improved to realize prompt investigation of cause when abnormality occurs in the equipment or the product, thereby realizing improvement in machine operation rate and rate of good product production.

The above-mentioned Patent Literatures 1 to 3 describe nothing regarding acquisition of data generated from production equipment forming a production line in order to store and utilize the data in association with product data. If a massive amount of data relating to the production equipment or the product can be utilized, improvement in productivity and quality of the product can be expected.

That is, a possibility emerges of realizing prompt investigation of the cause when a fault occurs in the product produced in the production equipment of the production line, and of realizing prompt investigation of the cause upon occurrence of a machine problem in the production equipment. Further, a possibility also emerges of maintaining equipment in good condition by predicting machine problems in advance and implementing remedies before the problems occur. Although this requires handling of massive amounts of data, the number of data that can be collected and accumulated by a general-purpose data logger is limited to only several to tens of the data. Massive amounts of data generated by production equipment have therefore been impossible to acquire and store. Moreover, the data are required not only to be stored, but also to be simultaneously sampled in time series and accumulated in association with each other, so that effective utilization of the data is desired.

In the method of producing a product according to the present invention, improvement in productivity can be realized by utilizing the data generated by the production equipment. That is, prompt analysis of the cause upon abnormality occurrence can be conducted by improved traceability of the defective product, and the improvement in the machine operation rate and the improvement in the rate of good product production can be realized.

In the apparatus for producing a product according to the present invention, the data relating to production can be utilized, and prompt analysis of the cause upon abnormality occurrence can be conducted by improved traceability of the production equipment, and the improvement in the machine operation rate and the improvement in the rate of good product production can be realized.

One preferable embodiment of the method of producing a product according to the present invention will be described below by taking a method of producing an absorbent article as one example with reference to drawings. First, one preferable example of a production apparatus will be described with reference to FIG. 1.

As shown in FIG. 1, a production apparatus 10 has a plurality of production lines 20, and a plant central processing unit 30 for controlling the production lines 20. The plant central processing unit 30 causes processing of "the number of products", "the number of good products", "an operating time", "a stopping time", "the number of stopping", "type number" and "the number of discharge for each sensor" for each production line 20. Each production line 20 has a line central processing unit 22 for controlling operation, stopping or the like of the production line, in a line network 21 connecting production equipment (not shown). Moreover, each production line 20 has an equipment central processing unit 23 for controlling each production equipment, a line display device 24 for displaying an operating state of the production line, and an inspection device 50 described later. Further, although not shown for all the production lines 20, a data collection unit 70 having a data collection network 25 is arranged in each line network 21 in each production line 20. In FIG. 1, the data collection unit 70 arranged in the line network 21 of the line No. XX is described as a representative. To each line network 21, the data collection network 25 of the data collection unit 70 arranged therein is connected. In addition, a detail of the data collection unit 70 will be described later. The above-described line network 21 is a network connecting the production equipment described later with each other, for example.

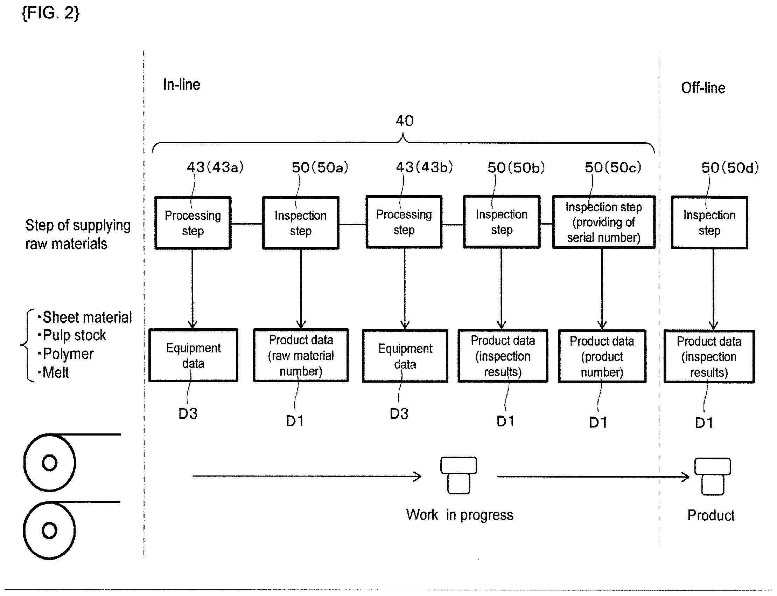

As shown in FIG. 2, production equipment 40 have a processing device 43 for performing a processing step, an inspection device 50 for performing an inspection step, and the like, which are arranged in in-line. The processing device 43, for example, includes a processing device 43A for processing a raw material, a processing device 43B for processing a work in progress, and the like. Equipment data D3 can be obtained from each processing device 43. Examples of the inspection device 50 in in-line include an inspection device 50a for acquiring the raw material number to allow the number to store in product data, an inspection device 50b for performing inspection after processing and an inspection device 50c for providing the product number. On the other hand, in off-line, the inspection device 50 for performing the inspection step in off-line is arranged. In this inspection step, as final inspection of the product, a shape, a dimension, mass and the like are inspected, for example. Product data D1 can be obtained from each inspection device 50. The work in progress corresponds thereto within the production equipment 40. The product data D1 includes the product data of not only a finished product but also the work in progress.

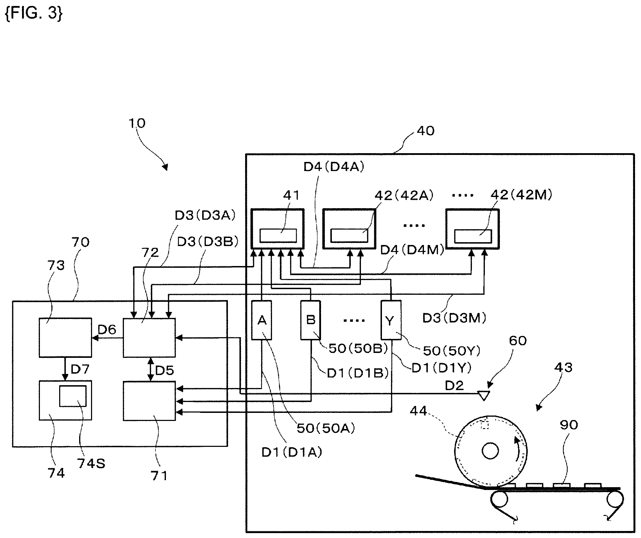

As shown in FIG. 3, the production apparatus 10 has the plurality of production lines 20 having the plurality of production equipment 40 for producing a product 90, and the plurality (for example, A to Y) of inspection devices 50 for inspecting the product 90 (work in progress) under production. That is, the product 90 includes the product of the finished product as well as the product of the work in progress.

The equipment data D3 (D3A, D3B, ..., D3M) obtained from the production equipment 40 is directly transmitted to a main central processing unit 72 of the data collection unit 70 from an equipment main control CPU 41 and an equipment auxiliary control CPU 42 (42A, 42B, ..., 42M) arranged within the production equipment 40. Moreover, equipment auxiliary data D4 (D4A, D4B, ..., D4M) obtained from the equipment auxiliary control CPU 42 is transmitted between the equipment auxiliary control CPU 42 (42A, 42B, ..., 42M) and the equipment main control CPU 41. The equipment auxiliary data D4 collected in the equipment main control CPU 41 can be transmitted to the main central processing unit 72 as the equipment data D3. In addition, in each figure, a communication path of various "data" is shown by an arrow for convenience. The same shall apply hereinafter. Moreover, a term "CPU" means a central processing unit.

The main central processing unit 72 causes reading of the data of the equipment main control CPU 41 or the equipment auxiliary control CPU 42 arranged within the production equipment 40, or writing of the data of the equipment main control CPU 41 or the equipment auxiliary control CPU 42 arranged therein. For communication between the equipment main control CPU 41 and the main central processing unit 72 or between the equipment auxiliary control CPU 42 and the main central processing unit 72, for example, LAN (Local Area Network) can be used as an ordinary communication method, for example. As LAN, for example, communication using Ethernet (registered trademark) is desirable. Accordingly, the equipment data D3 can be collected in the data collection unit 70 without significantly correcting software and hardware of the equipment main control CPU 41 or the equipment auxiliary control CPU 42. As described above, the product data D1 and the equipment data D3 are stored in the data collection unit 70 (data storing step).

Moreover, the production apparatus 10 has a sensor 60 for detecting a production pattern 44 of the processing device 43. The sensor 60 is configured of a proximity sensor, and a dog for operating this proximity sensor.

Further, the production apparatus 10 has the data collection unit 70 in which the product data D1 (D1A, D1B, ..., D1Y) obtained in the inspection device 50 and the production pattern data D2 detected in the sensor 60 are stored in association with each other. Specifically, for example, the product data D1 relating to a shape of the product 90 obtained in the inspection device 50 and the production pattern data D2 of the production pattern 44 of the processing device 43 in which the product shape detected in the sensor 60 is prepared are stored in the data collection unit 70 in association with each other. Both are thus associated with each other, and when abnormality is found in the product data D1, the production pattern data D2 in association with the product data D1 in which any abnormality is caused can be immediately retrieved. Accordingly, a place of the processing device 43, in which any abnormality is caused, can be easily found out.

The data collection unit 70 has an auxiliary central processing unit 71 and a main central processing unit 72. In the auxiliary central processing unit 71, the product data D1 is processed. Specifically, a data format (a communication standard, a file format and the like) of the product data D1 acquired in the inspection device 50 is transformed so as to be able to handle in the main central processing unit 72. Accordingly, if the product data D1 obtained from the inspection device 50 can be directly input into the main central processing unit 72, the auxiliary central processing unit 71 can be omitted.

Moreover, in the main central processing unit 72, the equipment data D3 indicating an equipment state of the production equipment 40, the auxiliary unit collection data D5 processed in the auxiliary central processing unit 71, and the production pattern data D2 are processed in association with each other.

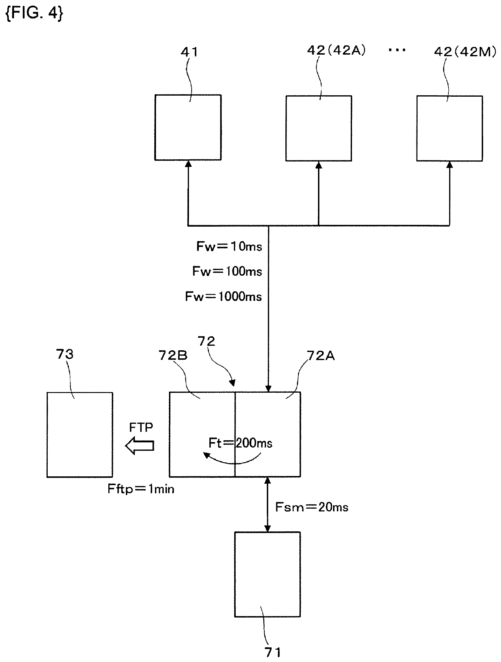

Specifically, as shown in FIG. 4, the main central processing unit 72 is mounted with two sets of main central processing CPUs 72A and 72B. In the first set of the main central processing CPU 72A, data of the equipment main control CPU 41, the equipment auxiliary control CPU 42 (42A, ..., 42M) and the auxiliary central processing unit 71 are obtained in batch. A sampling period at this time is adjusted to a period of time equal to or less than a processing period for each product.

The data obtained in the main central processing CPU 72A is transmitted to the main central processing CPU 72B in a period for each product. Thus, the data separately taken for each equipment can be arranged in the data in the same time series. At this time, the data is stored by using two kinds of triggers. A detail of data storage will be described later.

Thus, timing of abnormality occurrence can be traced by aligning the data in time series and collecting the aligned data. In the above-described main central processing unit 72, the data is collected in batch in the main central processing CPU 72A alone for time synchronization.

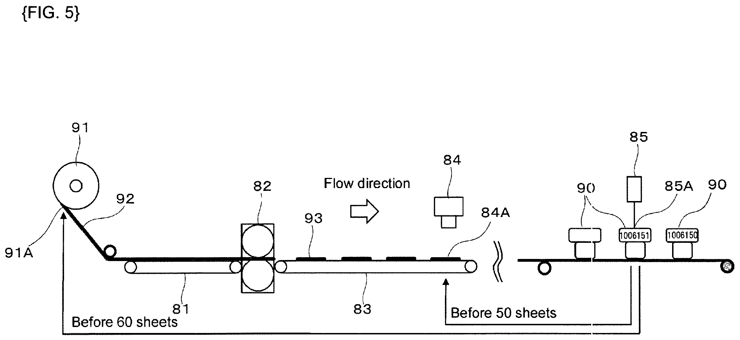

Specific association can be achieved by comparing the data with each other based on a distance between the steps (the number of processing). A step of associating the equipment data D3 with the product data D1 is performed based on the distance between the production steps, represented by the number of processing. For example, as shown in FIG. 5, a sheet 92 paid out from a sheet stock 91 is conveyed by a belt conveyor 81 and cut by a cutter 82. The cut sheets 93 are re-arranged (re-pitched) on the belt conveyor 83 by the belt conveyor 83 at a predetermined interval. Images of the re-pitched and cut sheets 93 are picked up by an image pickup device 84. Then, the cut sheets 93 are processed through various processing steps into a product 90. The product 90 proceeds to a serial number providing step, and is provided with a serial number by a serial number providing device 85. In the serial number providing step, a different serial number is printed on the product 90 for each product. Moreover, in the serial number providing step, the serial number may be printed on the product 90 in a continuous web state. Further, in the serial number providing step, the serial number may be printed on a package for packaging one or more products 90. A term "flow direction" in the figure means a conveyance direction of each sheet or each product 90.

When any other data resulting from the serial number is associated therewith, a distance between a serial number providing position 85A by the serial number providing device 85 and a position in another step is confirmed in advance. Specific examples of the position in another step include an image pickup position 84A by the image pickup device 84 in an image pickup step, and a stock position 91A, which is a position for paying out a paid-out sheet 92 of the sheet stock 91, in an illustrated example. Here, each distance is allowed to correspond to the number of cut sheets. As one example, the image pickup position 84A is at a 50th position from the serial number providing position 85A. The stock position 91A is at a 60th position from the serial number providing position 85A. The number of sheets corresponding to the above-described distance changes depending on the production apparatus. Thus, the distance from the serial number providing position 85A to the image pickup position 84A or the stock position 91A can be identified by allowing the distance to correspond to the number of sheets.

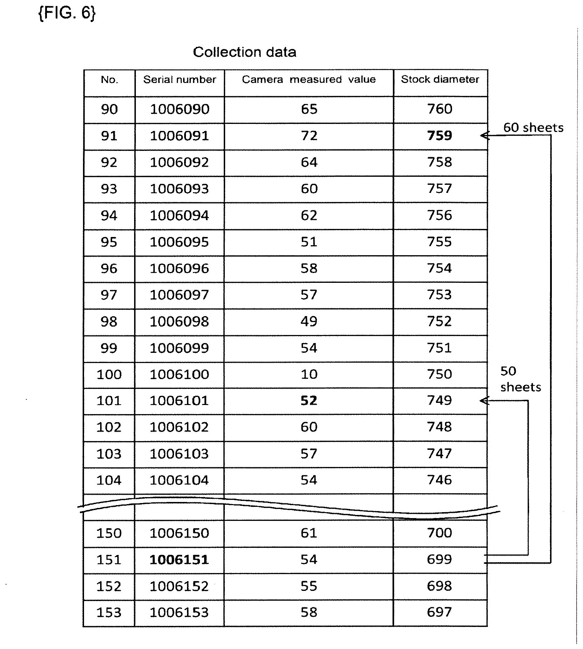

Accordingly, as shown in FIG. 6, collection data at timing of a serial number 1006151 is: a camera measured value = 54 pix. (pixel) and a stock diameter = 699 mm, but this data is not data in association with the serial number 1006151. The number of products between the serial number providing position 85A (see FIG. 5) and the image pickup position 84A (see FIG. 5) is 50. Accordingly, a camera measured value of 52 pix. in the image pickup position of No. 101, which is traced back by 50, corresponds to a value in association with the serial number 1006151. Moreover, a serial number in association with the stock position 91A (see FIG. 5) is 1006091 of No. 91, which is traced back by 60. This stock diameter value of 759 mm is a value in association with the serial number 1006151. Accordingly, the camera measured value and the stock value in association with the serial number 1006151 are 52 pix. and 759 mm, respectively. A term "camera measured value" described above means a scratch area value on a material surface determined based on image data picked up by the image pickup device 84 (see FIG. 5). A term "scratch area value" means a total area (pix.) of scratches recognized on the material surface. A unit of the camera measured value shown in FIG. 6 is pix. (pixel), and a unit of the stock diameter is mm.

Transmitted data from the main central processing CPU 72A is temporarily stored in the main central processing CPU 72B, and is transmitted to a database server 73 described later for each accumulation for a predetermined period of time. On the occasion, data communication is performed according to a file transfer protocol (FTP), and therefore the data can be transferred without stopping data logging of the main central processing CPU 72B.

One example of a transmission interval is shown below.

For example, when a processing speed is 300 sheets/min for absorbent articles as the product 90, more specifically, when a processing time per one sheet of the absorbent article is 200 ms, a reading period Fw from the main central processing CPU 72A to the equipment main control CPU 41 is set to 10 ms, 100 ms and 1000 ms, for example. Moreover, reading from the main central processing CPU 72A to the auxiliary central processing unit 71 is set to a period: Fsm = 20 ms, for example. Further, a transmission period Ft from the main central processing CPU 72A to the main central processing CPU 72B is set to a processing time of 200 ms per sheet by utilizing the trigger. A trigger interval is set to 200 ms, for example.

A transmission period Fftp from the main central processing CPU 72B to the database server 73 is set to 1 minute, for example.

Moreover, as shown in FIG. 3 or FIG. 4, the data collection unit 70 has the database server 73 in which main unit collection data D6 processed in the main central processing unit 72 is stored.

The above-described database server 73 is a server having database thereinside, in which a database control system is operated. For example, the server causes processing such as retrieval of the database in response to a request from an operator to perform operation of returning of processing results. A term "database control system" means software for operating and controlling the database necessary for constructing the database of a computer. Moreover, a term "database" means a file structure in which the data format is defined in advance, and the data can be integrally controlled.

Further, the data collection unit 70 has a display device 74 for displaying database data D7 stored in this database server 73. Specific examples of the display device 74 include a general display such as a liquid crystal display and an organic EL (organic electroluminescence) display.

Next, one preferable example of the method of producing a product will be described with reference to FIG. 3.

The method of producing a product according to the present invention has traceability, for each product 90, to the equipment data D3 and the equipment auxiliary data D4 of the production equipment 40 forming the production line 20 (see FIG. 1) for producing the product 90. The traceability is also referred to as trace capability. That is, the traceability means a traceable state from a production stage of the product 90 to a consumption stage (disposal stage).

In the present production method, the following steps are performed in focusing on one product line of the plurality of production lines. In addition, the following steps can also be performed on individual production lines of the total production lines.

First, a state of the product 90 is inspected in the inspection device 50 for each product 90 to acquire the product data D1 (product data acquisition step). For example, the product data D1 is acquired during performing processing in the production equipment 40. Accordingly, the production equipment 40 is associated with the inspection device 50. When the production equipment 40 has the plurality of inspection devices 50, the product data D1 (D1A, D1B, ..., D1Y) obtained by inspecting the state in each inspection device 50 (50A, 50B, ..., 50Y) is acquired. Specific examples of the state of the product 90 include a shape, a thickness and density. Specific examples of the above-described product data D1 include a position, an area, a dimension and density. Specific examples of the position include a traveling position of an elastic member of an absorbent article, a laminating position of the absorbent body and a product meandering position. A term "area" means an area in a planer view, an area of a cross section, or the like of a pattern of the product 90, obtained by processing the image obtained in the inspection device 50. A term "dimension" means a length, a width or a thickness of the pattern of the product 90, obtained by processing the image obtained in the inspection device 50, an angle formed by the patterns, or the like. Moreover, specific examples thereof include a shape, a thickness and basis weight of an absorbent body.

Next, the product data D1 is associated with the equipment data D3 indicating an equipment state of the production line 20 (data association step). Specific examples of the equipment data D3 include data of the number of products, an operating time, the number of stopping and an operating speed, as an operating state of the production equipment 40.

In addition thereto, specific examples thereof include data of a roll temperature, a stock diameter, a use shaft, a load ratio or the number of revolutions of a servomotor, timing of splicing, pattern operation information, an image measured value, a position of a pattern and a product counter. The total number of the data of the equipment as described above is about 1,000 to 2,000 as one example.

Specific examples of the roll temperature include data of a temperature of each processing roll for performing processing such as cutting, embossing and sealing, and data of a temperature of an anvil roll arranged with facing each processing roll.

Specific examples of the stock diameter include a current pay-off diameter of the stock.

As the use shaft, a using shaft among a plurality of shafts in which the same step is performed is specified.

The servomotor is a motor which is automatically actuated so as to follow a target value by applying, as a control amount, position data, direction data, posture data and the like of the product. Specific examples of the equipment data D3 of the servomotor include data of the number of revolutions (rotational speed) and a load ratio of a shaft. The number of revolutions changes depending on inside-winding or outside-winding when a speed of the stock paid out from the stock is fixed.

Specific examples of the timing of splicing include timing data of splicing immediately before splicing or immediately after the splicing.

Specific examples of the pattern operation information include positioning data of the pattern.

Specific examples of the image measured value include data of a position, an area, a shape or the like read from image processing data acquired by performing image processing of an image obtained in an inspection device in an image processing device (not shown) within the inspection device.

Specific examples of the position of the pattern include data repeated every x-th processing in a processing unit such as a fiber-stacking drum, a cutter and a side seal within a processing device for performing processing a plurality thereof within production equipment. X is taken as an integer of 2 or more. Specific examples include even number-th data.

Specific examples of the product counter include data of a production date, time of day, a plant line number, a lot number, a serial number, and a control number of a material by printing using a printer. Printing of the above-described items such as the serial number includes printing with colored ink and also printing with colorless ink. Specific examples of the colorless ink include UV ink which can be visualized by applying ultraviolet light thereto.

The product data D1 is associated with the equipment data D3 based on the distance between the production steps. Specifically, both are associated with other by allowing the lot number or the serial number of the product 90 to correspond to the equipment data D3 of the production equipment 40 in which the product 90 is processed for each product 90, for example. Further, individual product data D1 acquired in each inspection device 50 are associated with the equipment data D3 of the product pattern of the production equipment 40 in which a region from which individual product data D1 are acquired is prepared. At this time, a difference is produced between time at which individual product data D1 are acquired, and time at which the region of the product 90 from which individual product data D1 are acquired is processed in the production equipment 40, and therefore a temporal difference is preferably adjusted.

Then, the product data D1 and the equipment data D3 in association with each other are stored in the data collection unit 70 (associated data storing step).

Any product abnormality of the product data D1 is detected (judged) on the product 90 in progress in the production line 20 or the product 90 in which the steps to be implemented in the production line 20 are completed. Then, when any product abnormality occurs, product abnormality data D1n (not shown) in association with the product 90 in which any product abnormality is caused among the product data D1 is identified. The product data is traced based on the product abnormality data D1n identified. Here, equipment abnormality data D3n in association with the product deemed to be abnormal may be traced (abnormal product tracing step). Alternatively, both the equipment abnormality data D3n and the product abnormality data D1n, in association with the product deemed to be abnormal, may be traced. A term "trace" herein means tracing of the production process of the product 90 causing any abnormality, based on the product abnormality data D1n, as one example. Then, as a result of tracing the data, both or one of the product abnormality data D1n and the equipment abnormality data D3n is identified. Accordingly, the production step causing any product abnormality is further identified. A state of the production equipment 40 in which any abnormality occurs can be investigated from the equipment abnormality data D3n in the production equipment in the production step identified.

For example, an abnormal region of the production pattern 44 of the processing device 43 causing any product abnormality is identified by tracing the production process to acquire the equipment abnormality data D3n (not shown) (equipment abnormality data acquisition step).

The equipment abnormality data D3n is preferably visualized on a screen 74S of the display device 74 (visualizing step). A term "visualize" means one of control methods in a production site of the product. The term means that persons engaging in the production can specifically understand various activities in production of the product, such as production planning, production implementation, product evaluation and verification of problems of the product. For example, the term means that various activities described above are visualized on the screen of the display device. Moreover, in problem solving or improvement in the production, such a method is established as actively taking measures according to a production site level to achieve improvement or enhancement. Thus, the term "visualize" means that visualized problems are in a state in which judgement criteria on coping therewith are always shared within the production side, and the problems or issues are repeatedly improved. Specific examples of specifically visualized data include a machine operation rate, a rate of good product production, the number of machine stopping, a transition of the number of discharge of the defective product, and a time-series graph of data of a sensor measuring a shape of the product, a defect of the product or the like.

A visualized abnormal region in the production equipment 40 can be restored based on identification of the production step causing the above-described product abnormality (step of restoring a production equipment). Upon restoring the abnormal region, operation of the production equipment 40 is once stopped to restore the region to be restored in the production equipment 40. For example, machine parameters relating to an abnormal region in the production equipment 40 are adjusted to restore the abnormal region. At this time, whether or not the production equipment as a whole of the production line 20 having the production equipment to be restored is stopped is judged according to a place to be restored or a degree to be restored. In such restoration, although no feedback control is performed, as a method of restoring the abnormal region in the production equipment 40 according to visualization, for example, a method of correcting the machine parameters is shown. For example, a clearance or a roll temperature of an embossing roll can be adjusted while confirming a degree of an indentation using the image in an embossing step by using the embossing roll.

Moreover, the region can also be automatically restored by the feedback control depending on an object to be restored. For example, in adjusting the clearance, adjusting the temperature or the like in the above-mentioned embossing step, the feedback control can be made.

The above-described method of producing a product is preferably performed for each production line 20 (see FIG.1).

According to the above-described method of producing a product, the following working effect is produced.
(1) The product data D1 is acquired for each product 90, and the product abnormality data D1n indicating abnormality of the product 90 is detected, and any abnormality of the product can be judged. The equipment abnormality data D3n of the production equipment 40 in which any abnormality is caused can be acquired from the product abnormality data D1n. Thus, the traceability of the production line 20 is improved.
(2) A data collection function can be improved because the product data D1 is acquired for each product 90.
(3) Any abnormality of the production equipment 40 can be understood from the product data D1 n, which is abnormality data of the product 90, and therefore cause analysis of abnormality occurrence becomes prompt and easy.
(4) The equipment abnormality data D3n can be visualized, and thus the equipment data D3 of the production equipment 40 in which any abnormality occurs is displayed on the display device 74, and therefore coping when any abnormality occurs in the production site becomes prompt.

As described above, the cause of the abnormal product (also referred to as the defective product) of the product 90 can be promptly understood, and thus a defective place can be promptly restored. Moreover, the cause of defectiveness of the production line 20 can be visualized, and therefore the defective place of defective production equipment 40 can be promptly restored. Accordingly, the stopping time of the production line 20 can be reduced to improve the machine operation rate. Moreover, the cause of defectiveness is promptly investigated, and therefore occurrence of the defective product can be reduced to improve the rate of good product production.

Moreover, the defective place can be restored even without visualizing the cause. For example, even if neither numeric data nor a graph is displayed on the screen of the display device, abnormality occurrence can be noticed to the operator by an audio alarm, an electronic sound alarm or the like. Moreover, a staff who is not in the production site can be informed of any abnormality by using a communication means such as E-mail.

With respect to any product abnormality in the above-described production method, normal product data D1g (not shown) of the normal product is compared with the product data D1 of the product 90 prepared in the production line 20, and then a product having data different from the product data D1g of the normal product is detected as the abnormal product (abnormal product detection step).

Then, cause data D8 (not shown) causing any abnormality is extracted from the product abnormality data D1n of the abnormal product (cause data extraction step).

Based on this cause data D8, production pattern abnormality data D2n (not shown) of the equipment data D3 associated with the product data D1 is traced (abnormal product tracing step). Thus, a place of occurrence of the abnormal product of the product is associated with the equipment of abnormality occurrence.

In the product data D1 stored in the data collection unit 70, in addition to the product data D1 obtained by inspection in each inspection device 50, production data DP (not shown) of each product 90 is recorded in association with each product 90. Specific examples of the production data DP include a plant name, a line name, a production implementation date and a serial number.

Moreover, in the above-described method of producing a product, a statistical value can be calculated in a data collection step. As the statistical value, an average value, a standard deviation value and the like can be determined.

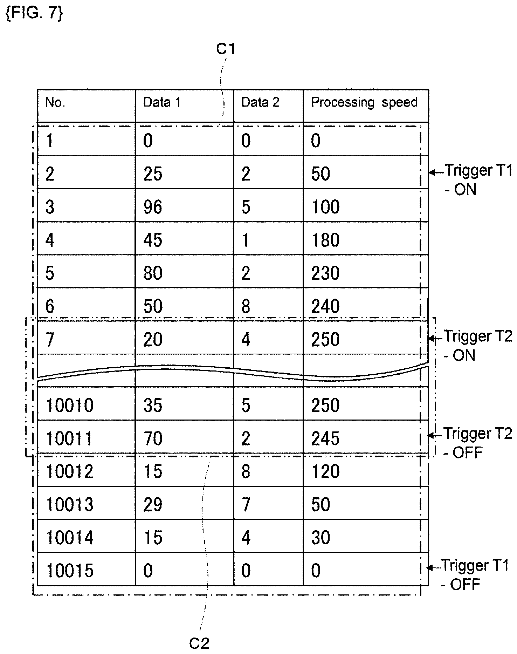

The statistical value is calculated in each step of the data collection step of the normal product and the data collection step of the abnormal product. On the occasion, the statistical value may be calculated without separating the good product (normal product) and the defective product (abnormal product). As shown in FIG. 7, the step has two sections in which statistical values are calculated. A first calculation section C1 is taken as a calculation section only for high-speed period data during high-speed operation (data during production). A second calculation section C2 is taken as a calculation section including low-speed period data and high-speed period data during low-speed and high-speed operation (data also including regulating operation). For example, when the product 90 is the absorbent article, if a processing speed of the absorbent article is 300 pieces/minute, the statistical value is calculated only when the speed is 300 pieces/minute during high-speed operation, and the statistical value is calculated when the speed is 50 pieces/minute or more during low-speed and high-speed operation.

Moreover, the statistical value is utilized in the following cases. For example, when the average value is significantly varied, the statistical value can represent that capacity of the equipment per se is significantly varied (for example, failed). Moreover, when the standard deviation is significantly varied, occurrence of abnormality which is rarely caused can be captured. Specific examples of the occurrence of abnormality which is rarely caused include poor processing caused by slight stain of the equipment or deterioration of a measuring sensor.

According to the standard deviation, behavior that cannot be captured by a variation of the average value is captured. Then, a possibility of early detecting any abnormality of the equipment is improved by using both the average value and the standard deviation.

Further, the above-described method of producing a product includes a step of confirming a correlation between image processing data D1IP (not shown) relating to an image processing inspection instrument in the product data D1, and the equipment data D3. The image processing inspection instrument is the inspection device for obtaining and inspecting the image among the inspection devices 50, and mainly has an image pickup device for picking up the image of an inspection area and an image processing device for data conversion processing of the image picked up in the image pickup device.

The step of confirming the above-described correlation includes a case of confirming the correlation in in-line, that is, a case of confirming the correlation in real time, and a case of confirming the correlation in off-line, that is, a case of confirming a detailed correlation after the data is collected.

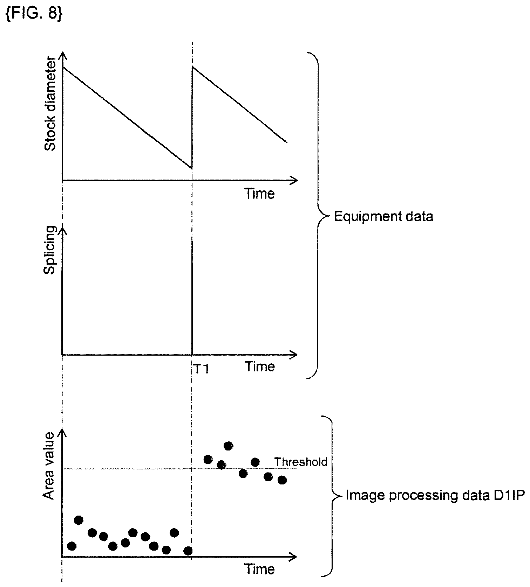

As shown in FIG. 8, the stock diameter is reduced over operation time, and when a level reaches the stock diameter necessary to be spliced, or when a splicing schedule time T1 is elapsed from start of operation, splicing has been carried out. Although no area value as usual is generated before splicing, an area value larger than a threshold is frequently generated after splicing. Occurrence of any abnormality is found by changing materials by splicing. Moreover, it can also be confirmed whether an inside of the stock is abnormal, or an outside thereof is abnormal by a correlation between the stock diameter and the area value.

In addition, a set value is registered in advance in the production equipment 40 of the production line 20, in addition to the product data D1. Examples of the set value include paper medium recording data in off-line.

The equipment data D3 is configured of sheet collection data D31 collected in the period for each product, long period collection data D32 collected in the period longer than the period of the sheet collection data D31, and short period collection data D33 collected in the period shorter than the period of the sheet collection data D31. An expression "collected in the period for each product" means that the equipment data D3 (sheet collection data D31) is taken for each product 90 processed in one of a plurality of the production equipment 40. Specific examples of the sheet collection data D31 include inspection data by an image inspection instrument because of inspection for each product. A term "sheet" herein means a sheet for each finished product, but also including a sheet for each in-process product, and a part of a semi-finished product corresponding to one product.

The long period collection data D32 is the equipment data D3 collected in the period longer than the period of the sheet collection data D31, and specific examples thereof include temperature data, and stock diameter data. The short period collection data D33 is the equipment data D3 collected in the period shorter than the period of the sheet collection data D31, and specific examples thereof include a pressure, a momentary load factor of a servomotor and a heater current value.

Thus, the present invention has advantages of increasing a processing speed to suppress stored data capacity by changing a storage period of the sheet collection data D31, the long period collection data D32 and the short cycle collection data D33.

Storage of collection data is started and stopped by the trigger. A term "trigger" means one of functions of the database control system, and a function of automatically activating processing designated in advance when any operation is applied to a table. A term "table" means a material in which elements such as data are arranged vertically and horizontally.

The stored data capacity can be suppressed by storing the data by the trigger. Moreover, at least two kinds of triggers are used for the trigger. In the trigger, a content of processing, activating conditions, timing of execution, and the like are designated and set. Specific examples thereof include designation of a speed more than a predetermined speed, designation of a threshold or less (%), designation of timing before and after defective product occurrence, and if the method of producing an absorbent article is applied, designation of timing before and after splicing.

Moreover, data in the case of different conditions can be compared by using a plurality of triggers. For example, as shown in FIG. 7 described above, two types of data including the data during high-speed operation (during normal production) and the data during low-speed operation are collected, and behavior during the high-speed operation and behavior during the low-speed operation are compared. The above-described high-speed operation ordinarily means operation at a maximum speed. The above-described low-speed operation means operation from start of processing to time immediately before reaching the high-speed operation, and from the time immediately after the high-speed operation to stopping of processing. Data collection during the low-speed operation is started by switching on (turning ON) a trigger T1. Data collection during the low-speed operation is performed from turning ON the trigger T1 until switching off (turning OFF) the trigger T1. Moreover, the data collection during the high-speed operation is started by switching on (turning on) a trigger T2, and the data collection during the high-speed operation is terminated by switching off (turning OFF) the trigger T2.

The above-described method of producing a product described in the present embodiment can be applied to a method of producing any other sheetform product, without being limited to the absorbent article as the product 90. The production method can also be applied to a method of producing a warmer, for example.

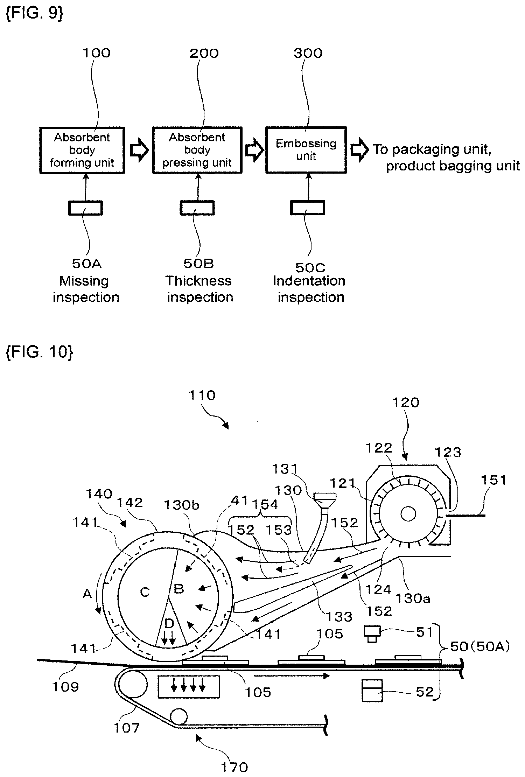

Next, one example when the above-described method of producing a product is applied to the method of producing an absorbent article will be described. As shown in FIG. 9, for example, the case where the method is applied to an absorbent body forming unit 100, an absorbent body pressing unit 200, and an embossing unit 300 will be described below. In the absorbent body forming unit 100, missing inspection of the pattern or the like is conducted by using an inspection device 50A. In the absorbent body pressing unit 200, thickness inspection is conducted by using an inspection device 50B. In the embossing unit 300, indentation inspection for inspecting an indentation state of an embossing pattern is conducted by using an inspection device 50C.

First, one example in which the production method is adopted in a fiber-stacking step as the absorbent body forming unit 100 will be described below with reference to FIG. 10.

As shown in FIG. 10, an absorbent body production apparatus 110 supplies an absorbent material 154 containing a fiber material, together with airflow, through an inside of a duct 130, and deposits the absorbent material 154 into a recess (hereinafter, also referred to as a fiber-staking recess) 141 arranged on a peripheral surface of a rotating drum 142 of a fiber stacker 140.

In a front stage of the production apparatus 110, the production apparatus 110 has a defibrator 120 for defibrating a pulp sheet 151 drawn from a pulp stock (not shown) to obtain the pulp fiber 152, and the duct 130 serving as a route for carrying and conveying on the airflow the pulp fiber 152 delivered from the defibrator 120.

The defibrator 120 has a casing 121, a rotating blade 122 disposed in the casing 121 to scratch an end portion of the pulp sheet 151. The casing 121 has an opening portion 123 provided in the casing 121 to introduce the pulp sheet 151 therefrom, and an opening portion 124 for discharging the pulp fiber 152 therefrom.

One end portion 130a of the duct 130 is connected to the opening portion 124 of the defibrator 120 and the other end portion 130b thereof partially covers the peripheral surface of the rotating drum 142.

In the rotating drum 142, for example, a plurality of fiber-stacking recesses 141 are formed on a circumferential surface at a predetermined interval. Toward the circumferential surface of this rotating drum 142, the absorbent material 154 (the pulp fiber 152, the absorbent polymer 153) (shown by an arrow, for convenience) conveyed inside the duct 130 is supplied and deposited into the fiber-stacking recess 141.

The absorbent body 105 stacked in the fiber-stacking recess 141 is used for the absorbent body of the absorbent article such as a sanitary napkin and an incontinence pad. Consequently, a shape of the above-described fiber-stacking recess 141 is determined according to the shape of the absorbent body 105. More specifically, the shape of the fiber-stacking recess 141 is determined such that a convex portion and a concave portion may be formed in sites necessary for the absorbent body 105. In addition, the shape of the fiber-stacking recess 141 is not limited thereto, and depth may be fixed, or the recesses 141 may be continuously formed along the peripheral surface of the rotating drum 142.

To the rotating drum 142, a suction fan (not shown) is connected, and partitioned space B in the rotating drum 142 is maintained in a negative pressure by working of the suction fan. A suction volume of the suction fan can be set by adjusting a frequency of an inverter (not shown), namely, a fiber-stacking suction frequency. The negative pressure in the space B generates the air flow in the duct 130 and the absorbent material 154 from the defibrator 120 is set in the flying state. At least a bottom portion of the individual fiber-stacking recesses 141 is configured by a mesh plate or the like as described above, and has many pores. While the individual fiber-stacking recesses 141 pass through the space B maintained in the negative pressure, the pores of the mesh plate function as suction holes. The space B is located on a side reverse to a part covered with the duct 130 in the rotating drum 142. The space B generates strong suction force in the fiber-stacking recess 141 passing through the part covered with the duct 130 to stack the absorbent material 154 in the fiber-stacking recess 141, and generates the air flow conveying the absorbent material 154 in the duct 130. In order to convey a stacked material or the absorbent body while it is stably retained in the fiber-stacking recess 141, a space C may be maintained in the negative pressure, and in this case, the space C is maintained in a negative pressure level lower than the level in the space B. Then, the airflow for conveying the absorbent material 154 flowing inside the above-described duct 130 is guided toward the peripheral surface of the rotating drum 142 by suction from the fiber-stacking recess 141 located above the space B.

Further, the production apparatus 110 has a conveying device 170 as a transfer conveying mechanism for releasing the absorbent body 105 from the fiber-stacking recess 141 and transferring the absorbent body 105 onto a covering sheet 109 composed of a water-permeable thin paper or nonwoven fabric. In addition, although not shown, a side portion of the covering sheet 109 below the absorbent body 105 may be folded to cover lower and upper surfaces of the absorbent body 105, and the production apparatus 110 may have a covering mechanism in which such operation is performed. Moreover, in addition to the covering sheet 109, a sheet may be separately supplied to cover the upper and lower surfaces of the absorbent body 105.

As the absorbent material 154, various kinds of materials to be used for the absorbent body of an absorbent article such as a sanitary napkin, a disposable diaper, or the like can be used without limitation, and the material 154 contains at least the fiber material. As the fiber material, for example, the pulp fiber obtained by defibrating the pulp sheet, and also a short fiber of a cellulose fiber such as a rayon fiber and a cotton fiber, a short fiber of a synthetic fiber of polyethylene, or the like is used. These fiber materials can be used alone in one kind or in combination of two or more kinds. Moreover, as the absorbent material 154, the absorbent polymer can be further used.

In the above-described fiber material, in addition to the above-mentioned pulp fiber 152, the synthetic fiber may be contained. The pulp sheet 151 can be in a state that the synthetic fiber is mixed therein, and the synthetic fiber can be mixed and supplied with the pulp fiber obtained by defibrating. In the production apparatus 110 shown in FIG. 10, the fiber is directly supplied from the defibrator 120 to the duct 130, but a defibrated pulp may be stored in a tank (not shown), and then may be supplied to the duct 130 therefrom. The synthetic fiber (short fiber) can also be supplied to this tank and mixed therein.

As described above, an absorbent body 105 is processed by stacking fibers by using the fiber stacker 140. Then, the absorbent body 105 released from the fiber-stacking recess 141 on the rotating drum 142 in the fiber stacker 140 is conveyed by being placed on a belt conveyor 107. In the conveying process therefor, a shape of the absorbent body 105 in which the fibers are stacked is inspected in the inspection device 50. In the inspection method, an image pickup device 51 and a lighting device 52 are used for the inspection device 50. The image pickup device 51 is arranged above the absorbent body 105 subjected to image pickup, and the lighting device 52 is arranged in a position facing the image pickup device 51 across the absorbent body 105. The absorbent body 105 is irradiated from a rear surface thereof by the lighting device 52, and an image of a shadow is picked up by the image pickup device 51. Accordingly, a material having light transmittance is used for the belt conveyor 107 in an area subjected to image pickup.

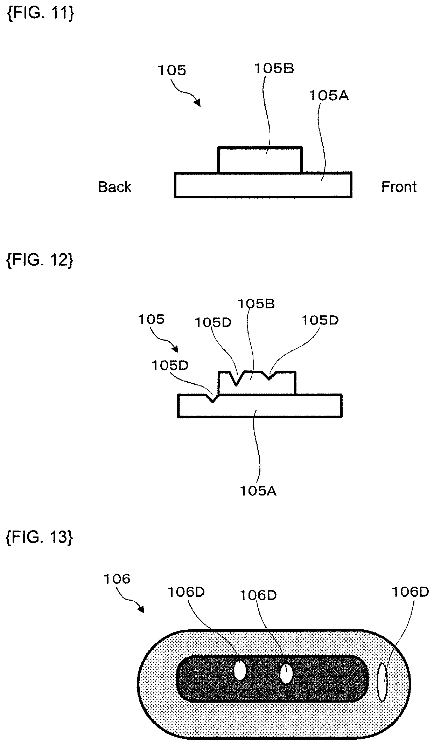

For example, as shown in FIG. 11, when the absorbent body 105 of a normal product is viewed by a cross section in a front-back direction, a rectangular crown portion 105B in the cross section is arranged on a rectangular whole portion 105A in the cross section. A term "front-back direction" means a direction in which, when a wearer wears the absorbent body 105, a side in which the absorbent body 105 is arranged on a front side of the wearer is referred to as "front", and a side in which the absorbent body 105 is arranged on a back side of the wearer is referred to as "back". Moreover, this front-back direction means a machine direction of the absorbent body 105.

In the case of the absorbent body 105, as shown in FIG. 12, when a concave portion 105D (or a convex portion) is observed on a surface of the whole portion 105A and a surface of the crown portion 105B, such an absorbent body 105 is deemed as the abnormal product (defective product).

When such a defect as described above is observed in the absorbent body 105, as shown in FIG. 13, an image of a concave portion 105D (see FIG. 12), which is a defective part, is picked up as a defective part image 106D in a pickup image 106 of the absorbent body. For example, when the thickness is excessively smaller than a surrounding, an image of the part is brightly picked up, and when the thickness is excessively larger than the surrounding, an image of the part is darkly picked up. A reason why the image is brightly picked up is that a light transmission amount in the part is larger than the amount in the surrounding, which means that the thickness of the absorbent body 105 is small. In such a case, clogging of a mesh plate is considered to be a cause, for example. Moreover, a reason why the image is darkly picked up is that the light transmission amount in the part is smaller than the amount in the surrounding, which means that the thickness of the absorbent body 105 is large. In such a case, mixing of foreign matters or increase of pulp is considered to be one of the causes, for example.

For example, an image processing camera system XG-8500L (trade name, manufactured by Keyence Corporation) is used as the above-described measuring instrument. As measurement data, a gray value and a missing area value of the absorbent body are taken.

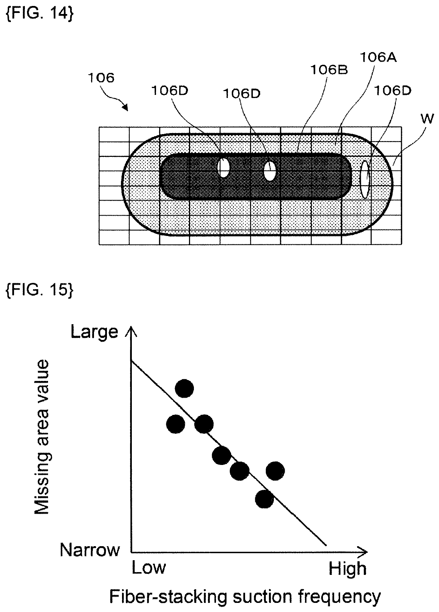

As shown in FIG. 14, the gray value of the absorbent body is determined by using an absorbent body image 106 picked up, dividing the image 106 into a mesh form in a plan view in a state in which the absorbent body 105 (see FIG. 12) picked up in the image is spread. In the absorbent body image 106, the light transmission amount of the crown portion 105B (see FIG. 12) in a central portion of the absorbent body 105 is reduced, and therefore such an image 106 results in the image darker than the whole portion image 106A around the crown portion image 106B. The whole portion 105A (see FIG. 12) is thinner than the crown portion 105B, and therefore the light transmission amount becomes larger than the amount of the crown portion 105B. As a result, the whole portion image 106A around the crown portion results in the image brighter than the crown portion image 106B. On such an image, for example, a plurality of windows are arranged in a lattice form, and a gray level (density) of an absorbent body part within the window is determined for each window.

Moreover, as shown in FIG. 14 mentioned above, the missing area value is determined by using the absorbent body image 106 picked up, and setting the plurality of windows W in the lattice form in a plan view in the state in which the absorbent body 105 (see FIG. 12) picked up in the image is spread. The absorbent body image 106 results in the image darker than the image of the whole portion image 106A around the crown portion image 106B in the similar manner as described above. On the other hand, the light transmission amount of the whole portion 105A becomes larger than the amount of the crown portion 105B, and therefore the whole portion image 106A results in the image brighter than the crown portion image 106B. Further, the defective part image 106D of the absorbent body picked up in the image results in the brightest image because the concave portion 105D (see FIG. 12) of the defective part is thinner than the crown portion 105B or the whole portion 105A, and the light transmission amount becomes large. Based on such an image, the area value of the part corresponding to the defective part image 106D is determined by performing binarization for each window W. A value obtained by totalizing the area values is taken as the missing area value.

FIG. 15 shows a relationship between the missing area value and the fiber-stacking suction frequency in a graph. As shown in FIG. 15, if the missing area is increased, the fiber-sticking suction frequency is decreased.

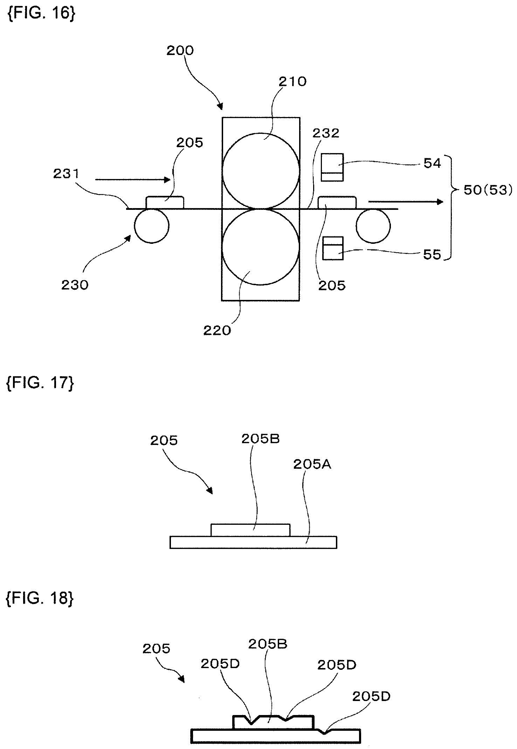

Pressing of the absorbent body is processing performed when the thickness of the absorbent body is reduced to increase fiber density, for example. As shown in FIG. 16, an absorbent body 205 is placed on a conveying belt 231 of a conveying device 230 and conveyed in an arrow direction. The absorbent body pressing unit 200 is provided in a conveying destination. In the absorbent body pressing unit 200, a pressure roll 210 for reducing the thickness of the absorbent body 205, and an anvil roll 220 having an interval from the pressure roll 210 are arranged in a position facing the pressure roll 210. The interval of peripheral surfaces of facing rolls between the pressure roll 210 and the anvil roll 220 is adjusted depending on the thickness of the absorbent body 205 in which the thickness thereof is reduced by pressurization. The absorbent body 205 which is interposed between the pressure roll 210 and the anvil roll 220 and pressurized as described above is reduced in the thickness, and placed on a conveying belt 232, and conveyed toward the next step. The conveying belts 231 and 232 are separated between an upstream side and a downstream side of the absorbent body pressing unit 200. In the conveying step, a height of the absorbent body 205 is measured by the inspection device 50. The inspection device 50 is configured of a displacement sensor 53. The displacement sensor 53 is divided into a flooding light projector 54 and a light receiver 55, and arranged in non-contact with the absorbent body 205 in a position facing the absorbent body 205 across the absorbent body 205 to be measured. As the displacement sensor 53, for example, LJ-V7300 (controller LJ-V 7000) (trade name) manufactured by Keyence Corporation is used. Measurement light is blue semiconductor laser light having a wavelength of 405 nm and a measurement width of 240 mm.

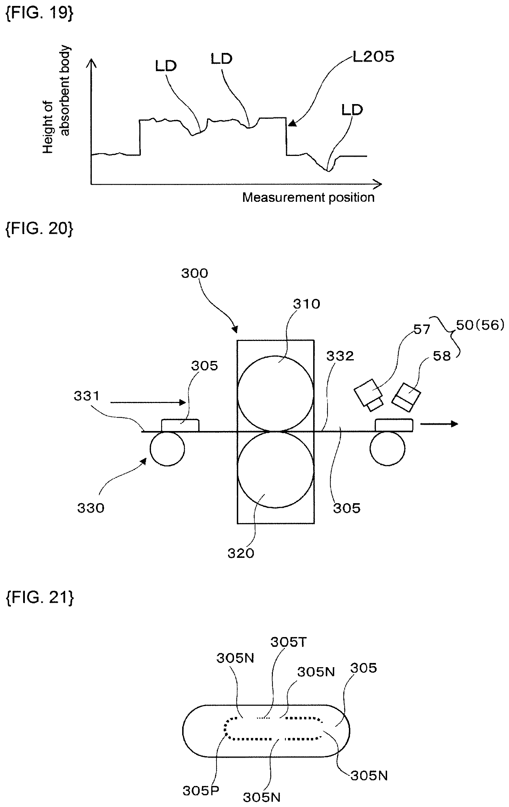

The height of the absorbent body 205 conveyed thereto in the front-back direction is measured by using the displacement sensor 53. For example, in the absorbent body 205 of the normal product as shown in FIG. 17, when viewed in the cross section in a longitudinal direction (machine direction), a rectangular crown portion 205B in the cross section is arranged on the rectangular whole portion 205 in the cross section. In addition, in FIGs. 17 and 18, description of hatching showing the cross section is omitted. Then, the height of the absorbent body 205, in the longitudinal direction, in which the crown portion 205B is arranged, is measured, thereby obtaining a surface shape in the longitudinal direction to determine a deformed shape of the absorbent body. As a result, a dent 205D is confirmed on the surface of the absorbent body 205 in the longitudinal direction as shown in FIG. 18 by the displacement sensor 53 (see FIG. 16). FIG. 19 shows results obtained by measurement by the displacement sensor 53. As shown in FIG. 19, a vertical axis represents a height of the surface of the absorbent body 205 measured in the displacement sensor 53, and a horizontal axis represents a position of the absorbent body 205 measured in the displacement sensor 53. In the measurement, a dent LD corresponding to the dent 205D (see FIG. 18) is confirmed on the surface of the absorbent body 205 in a line L205 indicating the height of the measured value.

Thus, in the case where the dent 205D is confirmed on the surface shape, such a case is deemed to be occurrence of the abnormal product (defective product), and the production line is stopped. That is, the production equipment 40 in which the abnormal product is caused is stopped to clean and check the processing unit in the production equipment 40. Moreover, the interval of a circumferential area between the pressure roll 210 and the anvil roll 220 and the clearance of the pressure roll 210 are checked and adjusted, and pressurizing force of the pressure roll 210 is checked and adjusted.

If the suction frequency of the fiber stacker is increased (a suction volume of the absorbent body is increased) as in the above-described method of producing a product, transferability is improved, a missing area of the absorbent body is reduced, and a processing state is improved. Therefore, the missing area of the absorbent body is detected to adjust the suction frequency so as to avoid occurrence of missing of the absorbent body.

For example, embossing of the absorbent body is processing for the purpose of improving leak-proofness, improving adhesion to an excretion portion by promoting raising in a portion facing the excretion portion, and the like.

As shown in FIG. 20, an absorbent body 305 is placed on a conveying belt 331 of a conveying device 330 and conveyed to an arrow direction. An embossing unit 300 for applying embossing to the absorbent body 305 is provided in the conveying destination. In the embossing unit 300, an embossing roll 310 for applying embossing to the absorbent body 305, and an anvil roll 320 having an interval from the embossing roll 310 are arranged in a position facing the embossing rolls 310. The interval of the peripheral surfaces of facing rolls between the embossing roll 310 and the anvil roll 320 is adjusted so that embossing may be prepared on the absorbent body by an embossing pattern. On the absorbent body 305 which is interposed between the embossing roll 310 and the anvil roll 320 described above and is pressurized by the embossing pattern, embossing is formed, and then the resulting material is placed on the conveying belt 332, and conveyed toward the next step. The conveying belts 331 and 332 are separated between the upstream side and the downstream side of the embossing unit 300. In the conveying step, an image of the absorbent body 305 is obtained by the inspection device 50. The inspection device 50 is an image processing camera system, and picks up an image of a surface of the absorbent body 305 to obtain a two-dimensional image. From the obtained two-dimensional image, a length, a position, a gray level and the like of an indentation prepared by embossing are measured. As a result of the measurement, as shown in FIG. 21, a part 305N having no embossing indentation is formed on the surface of the absorbent body 305. Moreover, a part 305T having a thin embossing indentation is also observed in several cases.

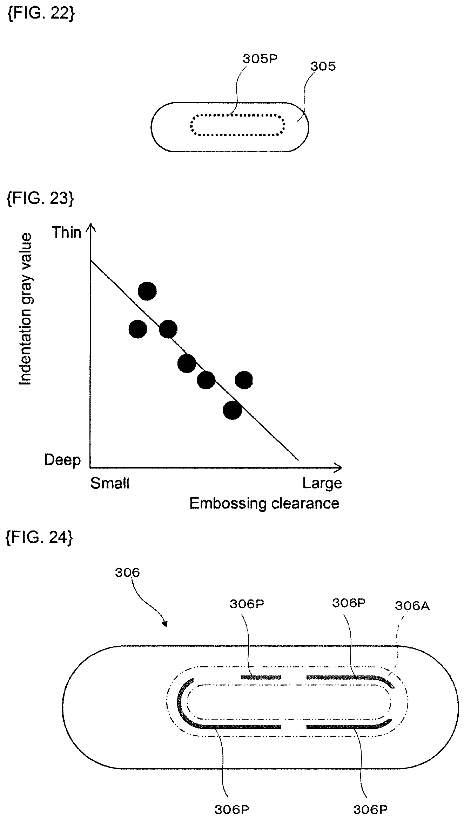

Moreover, as shown in FIG. 22, if a normal embossing pattern is formed, the embossing indentation 305P is arranged clearly and in aligned manner in an elliptical shape on the surface of the absorbent body 305.

Here, FIG. 23 shows a relationship between an indentation gray value of embossing and an embossing clearance. FIG. 23 shows that, accordingly as the embossing clearance decreases, the indentation gray value increases. That is, if the embossing clearance is decreased, the absorbent body is strongly pressed by the embossing pattern, and therefore the indentation gray value increases.

The above-described indentation gray value is determined by setting a plurality of windows (not shown) in the embossing portion and the surrounding thereof in a similar manner in FIG. 14 described above, and measuring density of the embossing image in each window. The window is preferably set so as to surround the embossing image portion 306P in FIG. 24 as described later. An inspection time can be shortened in comparison with a technology in which the window is set for the image as a whole, and processed and inspected.

An image processing camera system 56 in the inspection device 50 used for measurement is divided into an image pickup device 57 and a lighting device 58 for lighting an image pickup area, and both are arranged above the absorbent body 305 to be measured, and in non-contact with the absorbent body 305. Specific examples of the image processing camera system 56 include an image processing camera system CV-X 200 (trade name) manufactured by Keyence Corporation.

As shown in FIG. 24, density of an embossing image portion 306P and an embossing surrounding image portion 306A are measured from an absorbent body image 306 to determine an indentation gray value. Specifically, the indentation gray value is determined from a density gray scale value within each window mentioned above. Although not shown herein, if the embossing image portion 306P has no clearness relative to the embossing surrounding image portion 306A, the embossing image portion 306P has a whitish and pale image, and therefore the indentation gray value is decreased. Moreover, if the image portion has clearness, as shown in the figure, the portion has a blackish and dense image, and therefore the indentation gray value is increased. Then, the clearance of the embossing roll is adjusted based on the density in judging that no problem remains by evaluating product performance (leak-proofness, peeling, appearance or the like). If the clearance of the embossing roll is reduced (pressure is increased), the clearness of the embossing is improved. The clearance of the embossing roll is adjusted while sensing the clearness of the embossing from the pickup image. Specifically, the clearance of the embossing roll is adjusted based on a standard indentation gray value. Although not shown, with regard to the embossing clearance, a wedge is inserted between the embossing roll and the anvil roll, and the clearance is adjusted by an amount of insertion of the wedge.

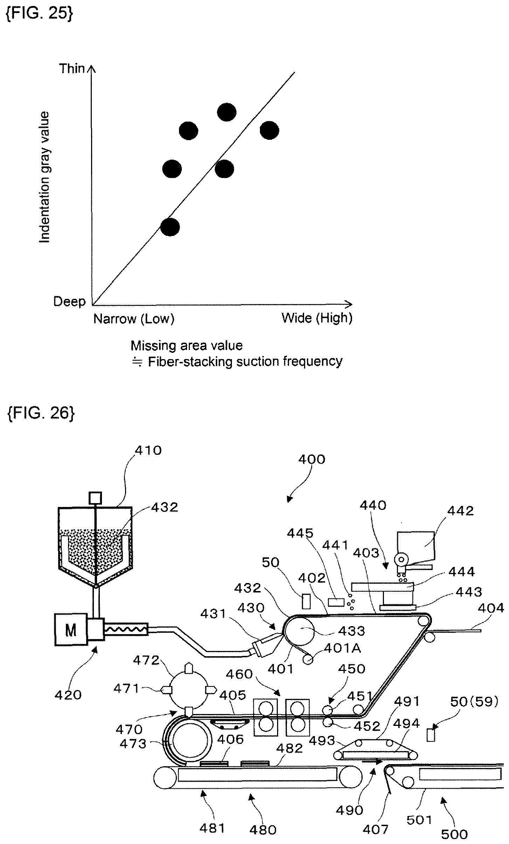

According to the above-described method of producing a product, different regions can be controlled by determining a causal relationship between inspection data in different steps. For example, a defect in absorbent body processing in an upstream step and a defect in embossing in a downstream step are correlated in several cases. For example, a cause of a poor indentation in embossing influences an absorbent body processing step in several cases. Specifically, as shown in FIG. 25, there is a relationship between the indentation gray value and the missing area value (fiber-stacking suction frequency). When the correlation as shown in FIG. 25 is obtained, the other can be controlled by controlling one. For example, in the absorbent body processing step, an indentation state can be improved if the suction frequency is controlled.

Thus, the causal relationship between the inspection data in the downstream step and the inspection data in the upstream step is established by combining the inspection data in the upstream step in the production line and the inspection data in the downstream step. Thus, a step different from the step in which any abnormality is found is controlled, which can be lead to improvement in the step in which any abnormality is found.

Moreover, not only two variables of the missing area value and the indentation gray value, but also the product data obtained by inspection by each inspection device can also be comprehensively analyzed. Such a technology can also be used for a processing region other than the absorbent body. For example, a poor thickness of the product can be detected from a poor product pack by taking a correlation between the thickness of the product and the product pack for packaging the product.

Next, a substantial portion of an apparatus for producing a heating element preferable for implementing the method of producing a heating element will be described with reference to FIG. 26.

As shown in FIG. 26, the apparatus for producing a heating element (hereinafter, abbreviated and referred to as a production apparatus) 400 has a coating unit 430, an electrolyte adding unit 440, and a laminating unit 450. The production apparatus further has a slit, incision processing unit 460, a first cutting unit 470, a re-pitching unit 480, a discharging unit 490 (flight conveyor 491), and a covering unit 500.

A coating material 432 that is adjusted in advance by an adjusting device (not shown) and accumulated in a storage tank 410 is fed to the coating unit 430 by a feeding pump 420.

In the present embodiment, in the coating unit 430, the coating material 432 is coated onto a first base material sheet 401 of a long belt body which is paid out from the stock roll 401A and is conveyed along the longitudinal direction to coat a heating element layer 403 (coating step).

The coating unit 430 has a die coater 431 for coating the coating material 432. Moreover, the first base material sheet 401 of the long belt body which is paid out from the stock roll 401A is conveyed by a coating roll 433 to a position facing a coating material discharging port of the die coater 431. The coating material 432 is coated onto one surface of the first base material sheet 401 by the die coater 431, and a coating material layer 402 as a heating element layer 403 is arranged.

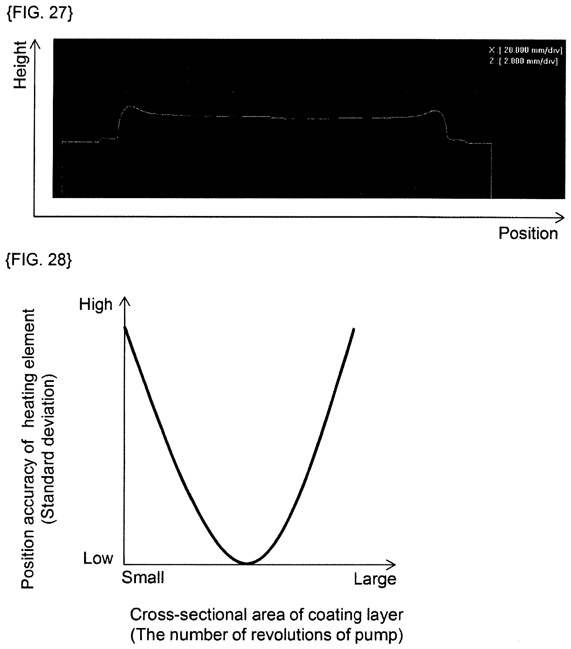

As described above, a cross-sectional area of the coating material layer 402 is measured by the inspection device 50 in a state in which the coating material layer 402 serving as the heating element layer 403 is coated onto the first base material sheet 401. The inspection device 50 is an optical surface shape measurement device to measure a surface shape of the coating material layer 402 in the conveying direction of the first base material sheet 401. FIG. 27 shows results of measuring the coating material layer 402 by the inspection device 50. As shown in FIG. 27, a vertical axis represents a height of the coating material layer 402 measured by the inspection device 50, and a horizontal axis represents a position of the coating material layer 402 in a direction (crosswise direction) crossing the longitudinal direction as measured by the inspection device 50. From the measurement results shown in FIG. 27, the cross-sectional area is determined by integrating data on the surface shape obtained by measurement. The obtained cross-sectional area is stored as the product data D1. In association therewith, a feed amount of the coating material 432 per unit time by using the feeding pump 420 is stored as the equipment data D3. The product data D1 and the equipment data D3 are stored in the data collection unit 70 in association with each other.

Then, when any product abnormality is detected on a product in progress in the production line or a product in which steps to be implemented in the production line are completed, the equipment data D3 is traced based on the product abnormality data D1n of the product in which any product abnormality is caused. For example, the equipment data D3 such as cutting timing using a rotary die cutter 472, a conveying speed by a conveying belt 482 and a suction pressure of a suction box 494 are traced based on the product abnormality data D1n. The abnormal region of the production pattern in the production equipment in which any product abnormality is caused is identified by tracing to obtain equipment abnormality data D3n. As a result, when the values are required to be adjusted, the production equipment 40 are stopped to adjust equipment in necessary places.

In the electrolyte adding unit 440, an electrolyte 441 is spread. For example, the electrolyte 441 is spread from a screw feeder 442 onto the coating material layer 402 through a trough 444 having an electrolyte mass sensor 443. A spread amount thereof is measured by an electrolyte spread amount sensor 445. Powder thereof is used as the electrolyte 441. Moreover, the first base material sheet 401 after being coated is conveyed from the coating unit 430 to the electrolyte adding unit 440 by a conveying device including the coating roll 433. Then, the electrolyte 441 is spread onto the coating material layer 402 toward a coated surface of the first base material sheet 401 into the heating element layer 403.

An electrolyte concentration preferable for heat generation can be secured in the heating element layer 403 by adding the electrolyte 441. Moreover, the electrolyte 441 in powder is dissolved by moisture contained in the coating material layer 402 and the first base material sheet 401. Further, a second base material sheet 404 is fed so as to be adhered on a side of the heating element layer 403, and sent to the laminating unit 450. In addition, the electrolyte may be powder or an aqueous solution.

As a result, moisture in the heating element layer 403 is absorbed and held in the second base material sheet 404, and a moisture content and the electrolyte concentration in the heating element layer 403 become preferable.

In the present embodiment, in the laminating unit 450, the first base material sheet 401 and the second base material sheet 404 are laminated across the heating element layer 403 (lamination step).

In the laminating unit 450, the heating element layer 403 formed on the first base material sheet 401 is laminated onto the first base material sheet 401 and the second base material sheet 404 by being interposed between nip rolls 451 and 452.

Subsequently, in a slit, incision processing unit 460, an incision processing step is performed in which a plurality of incisions are formed to bond the first base material sheet 401 and the second base material sheet 404.

The slit, incision processing unit 460 is a unit for forming incisions (perforations) and slits (not shown) in the longitudinal direction of the first base material sheet 401, namely, the conveying direction of the first base material sheet 401.

As described above, a heating element continuous body 405 formed of a continuous long object is prepared. Then, the heating element continuous body 405 is cut over a direction (crosswise direction) crossing the longitudinal direction in a first cutting unit 470 (cutting step). The first cutting unit 470 has the rotary die cutter 472 having a cutter blade 471 on a peripheral surface and an anvil roll 473. The heating element continuous body 405 is cut by passing between the rotary die cutter 472 and the anvil roll 473, and a plurality of sheets of heating elements 406 are obtained. The heating element 406 being cut is conveyed to the re-pitching unit 480 and received by a conveyor 481.

The heating element continuous body 405 is cut in the crosswise direction of the heating element continuous body 405. For example, the heating element continuous body 405 can be cut linearly over the crosswise direction of the heating element continuous body 405. Alternatively, the heating element continuous body 405 can be cut so that a cutting line may draw a curve.

The heating element 406 formed into the sheet is placed on the conveying belt 482 of the conveyor 481 arranged in the re-pitching unit 480. The conveying speed by the conveying belt 482 becomes higher than a circumferential speed of the anvil roll 473 installed in the first cutting unit 470. As a result, a distance between the heating elements 406 adjacent to front-back in the conveying direction is extended, and the heating elements 406 are reinstalled at a predetermined distance.

In the conveyor 481, the heating element 406 is ordinarily conveyed while being sucked on a side of the conveyor 481. In order to prevent the heating element 406 from dropping in a former stage of the flight conveyor 491, suction of the conveyor 481 in a latter stage may be stopped in conveyance of the heating element 406 in a next flight conveyer 491. Moreover, in the above-described conveyer 481, an interval of the heating element 406 in the crosswise direction is also widened. As such a re-pitching mechanism, a conventionally known mechanism can be used without particular limitation. In addition, a term "front-back in the conveying direction" means the upstream side and the downstream side in the conveying direction.

The heating element 406 which is re-pitched and widened in the crosswise direction is conveyed to the discharging unit 490. The discharging part 490 has the flight conveyor 491. The heating element 406 is conveyed in a state of being suspended by the flight conveyor 491. In order to realize the conveyance in such a state, a suction box 494 is installed inside a circular path in a position of the region directing downward. The heating element 406, which is an object to be conveyed, is conveyed in the above-described region by suction in a state of being sucked and supported on a support surface of an endless belt 493 by activating the suction box 494.

The heating element 406 passed through the discharging unit 490 is transferred to a conveyor 501 of the covering unit 500. In the covering unit 500, the heating element 406 as a whole is covered with a first covering sheet and a second covering sheet 407 (not shown).

Before being covered with the first covering sheet, the inspection device 50 for detecting a position of the heating element 406 is arranged. This inspection device 50 is configured of an image pickup device 59 and an image processing unit (not shown). The image of the heating element picked up in the image pickup device 59 is subjected to image processing by the image processing unit to detect the position of the heating element 406 to acquire the product data D1. In association therewith, for example, cutting timing using the rotary die cutter 472, the conveying speed by the conveying belt 482, the suction pressure of the suction box 494 and the like in the production apparatus 400 as the production equipment are stored, as the equipment data D3, in an equipment main processing unit 41 (see FIG. 3). The product data D1 and the equipment data D3 are stored in the data collection unit 70 in association with each other in a similar manner with reference to FIG. 3 as described above.

Then, when any product abnormality is detected on the product in progress in the production line or the product in which the steps to be implemented in the production line are completed, the equipment data D3 is traced based on the product data D1 of the product in which any product abnormality is caused. For example, based on the product data D1, the equipment data D3 such as the cutting timing using the rotary die cutter 472, the conveying speed by the conveying belt 482 and the suction pressure of the suction box 494 is traced, and associated with each other. The abnormal region of the production pattern in the production equipment in which any product abnormality is caused is identified by tracing to obtain the equipment abnormality data D3n. As a result, when the values are required to be adjusted, the production equipment (corresponding to the production equipment 40 in FIG. 3) are stopped to adjust the equipment in the necessary places.

When no adjustment is required, the above-described heating element 406 is covered with the second covering sheet 407 on a side on which the heating element layer 403 is not arranged. Then, the heating element 406 covered therewith is conveyed to a sealing unit (not shown) by the conveyor 501 while keeping a state covered with the first covering sheet and the second covering sheet 407.

Each heating element 406 is continuously covered with the first covering sheet and the second covering sheet 407 by the sealing unit to obtain a heating tool continuous body in a state in which a plurality of heating tools are connected in one direction. As the first covering sheet and the second covering sheet 407, for example, the same material as the material described in JP-A-2012-000344 ("JP-A" means unexamined published Japanese patent application), JP-A-2012-000345 or the like can be used. In a second cutting unit (not shown), the heating tool continuous body is cut across the crosswise direction between the heating elements adjacent to each other. The second cutting unit has a rotary die cutter and an anvil roll facing the cutter. The heating tool continuous body passes between both members, thereby being cut to obtain an objective heating tool (not shown).

As described above, in the next step (not shown), this heating tool is hermetically housed into a packaging bag having oxygen barrier properties.

Oxidizable metal is used as a raw material of the above-described coating material 432, and specific examples of the oxidizable metal include powder of iron, aluminum, zinc, manganese, magnesium, and calcium. Iron powder is preferably used.

In the above-described method of producing a heating element, the coating material 432 is fed to the coating unit 430 by the feeding pump 420. There is a correlation in which the feed amount of the coating material 432 is substantially proportional to the number of revolutions of the feeding pump 420, and if the number of revolutions of the pump is increased, cutting of the heating element continuous body 405 is improved. However, if the number of revolutions of the pump is excessively increased, a thickness of the coating layer 402 increases, and therefore cutting of the heating element continuous body 405 is deteriorated. A relationship between the cross-sectional area of the coating layer 402 to be measured by the inspection device 50 and position accuracy of the heating element to be measured by the image pickup device 59 is as shown in FIG. 28. Accordingly, the position accuracy of the heating element can be improved by controlling the number of revolutions of the feeding pump 420 within the specification range of the coating amount of the coating material 432. The position accuracy of the heating element is represented by a standard deviation of the interval between the heating element and the heating element, and means cutting accuracy of the heating element continuous body 405.

With respect to the above-mentioned embodiments and aspects, the present invention further discloses the following embodiments.
<1> A method of producing a product through a plurality of production steps, containing:
   a step of acquiring equipment data of production equipment for producing the product;
   a step of acquiring product data from the product;
   a step of storing the equipment data and the product data in a data collection unit;
   a step of associating the equipment data with the product data;
   a step of judging abnormality of the product data;
   a step responsive to abnormality occurring in the product for identifying one or both of equipment abnormality data and product abnormality data associated with the product judged to be abnormal; and
   a step of further identifying the production step causing the product abnormality by the step of identifying the abnormality data.
<2> The method of producing a product as described in the above item <1 >, containing:
   a step of judging abnormality of the product after producing the product;
   a step of identifying, when abnormality occurs in the product, both or one of the equipment abnormality data and the product abnormality data in association with the product deemed to be abnormal product; and
   a step of further identifying the production step causing any product abnormality by the step of identifying the data.
<3> The method of producing a product as described in the above item <1> or <2>, containing:
   a step of acquiring the equipment abnormality data on production equipment in a production step causing any product abnormality; and
   a step of visualizing any product abnormality data of the product deemed to be abnormal and the equipment abnormality data on a screen of a display device.
<4> The method of producing a product as described in any one of the above items <1> to <3>, wherein the product proceeds to a serial number providing step, and is provided with a serial number by a serial number providing device.
<5> The method of producing a product as described in the above item <4>, wherein, in the serial number providing step, a different serial number is printed on each product.
<6> The method of producing a product as described in the above item <4>, wherein, in the serial number providing step, the serial number is printed on the product in a continuous web state.
<7> The method of producing a product as described in the above item <4>, wherein, in the serial number providing step, the serial number is printed on a package for packaging one or more products.
<8> The method of producing a product as described in any one of the above items <1> to <7>, wherein a step of associating the equipment data with the product data is performed based on the distance between the production steps.
<9> The method of producing a product as described in any one of the above items <1> to <8>, containing a step of restoring a production equipment in a production step identified by the step of identifying the production step.
<10> The method of producing a product as described in the above item <9>, wherein in the step of restoring a production equipment, an abnormal region in the production equipment is automatically restored by feedback control.
<11> The method of producing a product as described in the above item <9> or <10>, wherein in the step of restoring a production equipment, the production equipment is stopped to restore an abnormal region by adjusting machine parameters relating to the abnormal region.
<12> The method of producing a product as described in any one of the above items <1> to <11>, containing the steps of:
   comparing product data of a normal product with product data of the product prepared in the production steps, to detect a product, as an abnormal product, having data different from the product data of the normal product;
   extracting cause data causing any abnormality from the product data of the abnormal product; and
   tracing production pattern data of the equipment data associated with the product data based on the cause data.
<13> The method of producing a product as described in any one of the above items <1> to <12>,
   wherein the product is an absorbent article, and
   wherein production data of the absorbent article is recorded in association with the absorbent article in the product data stored in the data collection unit.
<14> The method of producing a product as described in any one of the above items <1> to <13>, wherein the statistical value is calculated in each step of a data collection step of the normal product and a data collection step of the abnormal product.
<15> The method of producing a product as described in any one of the above items <1> to <14>, containing a step of confirming a correlation between image processing data relating to an image processing inspection instrument in the product data, and the equipment data.
<16> The method of producing a product as described in the above item <15>, wherein in the step of confirming a correlation, the correlation is confirmed in in-line.
<17> The method of producing a product as described in the above item <15>, wherein in the step of confirming a correlation, the correlation is confirmed in off-line.
<18> The method of producing a product as described in any one of the above items <1> to <17>, wherein the data collection unit has a main central processing unit and an auxiliary central processing unit.
<19> The method of producing a product as described in any one of the above items <1> to <18>, wherein a set value is registered in advance in the production equipment, in addition to the product data.
<20> The method of producing a product as described in any one of the above items <1> to <19>, wherein the equipment data is configured of:
   sheet collection data collected in the period for each product;
   long period collection data collected in the period longer than the period of the sheet collection data; and
   short period collection data collected in the period shorter than the period of the sheet collection data.
<21> The method of producing a product as described in any one of the above items <1> to <20>, wherein a serial number which identify the product is printed using colored ink or colorless ink.
<22> The method of producing a product as described in any one of the above items <1> to <21>, wherein the product is an absorbent article.
<23> The method of producing a product as described in any one of the above items <1> to <22>, wherein the product is a sheet-form product.
<24> An apparatus for producing a product, by which the product is produced correspondingly to a plurality of production steps, containing:
   a plurality of pieces of production equipment by which the product is produced;
   a plurality of inspection devices by which the product is inspected;
   a sensor for detecting a production pattern possessed by the production equipment; and
   a data collection unit in which product data obtained by the inspection devices and production pattern data detected by the sensor are stored in association with each other,
   wherein the data collection unit has:
   an auxiliary central processing unit in which the product data is processed,
   a main central processing unit in which mutually associated equipment data indicating equipment states of the production equipment, auxiliary unit collection data processed in the auxiliary central processing unit and the production pattern data are processed, and
   a database server in which main unit collection data processed in the main central processing unit are stored.
<25> The apparatus for producing a product as described in the above item <24>, wherein the auxiliary central processing unit transforms a data format of the product data acquired in the inspection device so as to be able to handle in the main central processing unit.

Having described our invention as related to this embodiments, it is our intention that the invention not be limited by any of the details of the description, unless otherwise specified, but rather be construed broadly within its spirit and scope as set out in the accompanying claims.

This application claims priority on Patent Application No. 2016-229251 filed in Japan on November 25, 2016, Patent Application No. 2017-021494 filed in Japan on February 8, 2017, and Patent Application No. 2017-219350 filed in Japan on November 14, 2017, each of which is entirely herein incorporated by reference.

### DESCRIPTION OF SYMBOLS

- 10: Production apparatus
- 20: Production line
- 21: Line network
- 22: Line central processing unit
- 23: Equipment central processing unit
- 24: Line display device
- 25: Data collection network
- 30: Plant central processing unit
- 40: Production equipment
- 41: Equipment main control CPU
- 42, 42A, ..., 42M: Equipment auxiliary control CPU
- 43, 43A, 43B: Processing device
- 44: Production pattern
- 50, 50A, 50B, ..., 50Y, 50a, 50b, 50c: Inspection device
- 51, 57, 59: Image pickup device
- 52, 58: Lighting device
- 53: Displacement sensor
- 54: Flooding light projector
- 55: Light receiver
- 56: Image processing camera system
- 60: Sensor
- 70: Data collection unit
- 71: Auxiliary central processing unit
- 72: Main central processing unit
- 73: Database server
- 74: Display device
- 81, 83: Belt conveyor
- 82: Cutter
- 84: Image pickup device
- 84A: Image pickup position
- 85: Serial number providing device
- 85A: Serial number providing position
- 90: Product
- 91: Sheet stock
- 91A: Stock position
- 92: Sheet paid out
- 93: Cut sheet
- 100: Absorbent body forming unit
- 105: Absorbent body
- 105D: Concave portion
- 106: Absorbent body image
- 106D: Defective part image
- 107: Belt conveyor
- 110: Production apparatus
- 120: Defibrator
- 121: Casing
- 122: Rotating blade
- 123, 124: Opening portion
- 130: Duct
- 130a: One end portion
- 130b: Other end portion
- 140: Fiber stacker
- 141: Fiber-stacking recess
- 142: Rotating drum
- 151: Pulp sheet
- 152: Pulp fiber
- 153: Absorbent polymer
- 154: Absorbent material
- 200: Absorbent body pressing unit
- 205: Absorbent body
- 205A: Whole portion
- 205B: Crown portion
- 205D: Dent
- 210: Pressure roll
- 220: Anvil roll
- 230: Conveying device
- 231, 232: Conveying belt
- 300: Embossing unit
- 305: Absorbent body
- 305P: Embossing indentation
- 305N: Part having no embossing indentation
- 305T: Part having thin embossing indentation
- 306: Absorbent body image
- 306P: Embossing image portion
- 306A: Embossing surrounding image portion
- 310: Embossing roll
- 320: Anvil roll
- 330: Conveying device
- 331, 332: Conveying belt
- 400: Apparatus for producing heating element
- 401: First base material sheet
- 401A: Stock roll
- 402: Coating material layer
- 403: Heating element layer
- 404: Second base material
- 405: Heating element continuous body
- 406: Heating element
- 410: Storage tank
- 420: Feeding pump
- 430: Coating unit
- 431: Die coater
- 432: Coating material
- 433: Coating roll
- 440: Electrolyte adding unit
- 441: Electrolyte
- 442: Screw feeder
- 443: Electrolyte mass sensor
- 444: Trough
- 445: Electrolyte spread amount sensor
- 450: Laminating unit
- 451, 452: Nip roll
- 460: Slit, incision processing unit
- 470: First cutting unit
- 471: Cutter blade
- 472: Rotary die cutter
- 480: Re-pitching unit
- 481: Conveyor
- 482: Conveying belt
- 490: Discharging unit
- 491: Flight conveyor
- 493: Endless belt
- 494: Suction box
- 500: Covering unit
- D1, D1A, D1B, ..., D1Y: Product data
- D2: Production pattern data
- D3, D3A, D3B, ..., D3M: Equipment data
- D4, D4A, D4B, ..., D4M: Equipment auxiliary data
- D5: Auxiliary unit collection data
- D6: Main unit collection data
- D7: Database data
- LD: dent
- L205: Line

## Claims

1. A method of producing a product through a plurality of production steps, comprising:
a step of acquiring equipment data of production equipment for producing the product;
a step of acquiring product data from the product;
a step of storing the equipment data and the product data in a data collection unit;
a step of associating the equipment data with the product data;
a step of judging abnormality of the product data;
a step responsive to abnormality occurring in the product for identifying one or both of equipment abnormality data and product abnormality data associated with the product judged to be abnormal; and
a step of further identifying the production step causing the product abnormality by the step of identifying the abnormality data.

2. The method of producing a product according to Claim 1, comprising:
a step of judging abnormality of the product after producing the product;
a step of identifying, when abnormality occurs in the product, both or one of the equipment abnormality data and the product abnormality data in association with the product deemed to be abnormal product; and
a step of further identifying the production step causing any product abnormality by the step of identifying the data.

3. The method of producing a product according to Claim 1 or 2, comprising:
a step of acquiring the equipment abnormality data on production equipment in a production step causing any product abnormality; and
a step of visualizing any product abnormality data of the product deemed to be abnormal and the equipment abnormality data on a screen of a display device.

4. The method of producing a product according to any one of Claims 1 to 3, wherein the product proceeds to a serial number providing step, and is provided with a serial number by a serial number providing device.

5. The method of producing a product according to Claim 4, wherein, in the serial number providing step, a different serial number is printed on each product.

6. The method of producing a product according to Claim 4, wherein, in the serial number providing step, the serial number is printed on the product in a continuous web state.

7. The method of producing a product according to Claim 4, wherein, in the serial number providing step, the serial number is printed on a package for packaging one or more products.

8. The method of producing a product according to any one of Claims 1 to 7, wherein a step of associating the equipment data with the product data is performed based on the distance between the production steps.

9. The method of producing a product according to any one of Claims 1 to 8, comprising a step of restoring a production equipment in a production step identified by the step of identifying the production step.

10. The method of producing a product according to Claim 9, wherein in the step of restoring a production equipment, an abnormal region in the production equipment is automatically restored by feedback control.

11. The method of producing a product according to Claim 9 or 10, wherein in the step of restoring a production equipment, the production equipment is stopped to restore an abnormal region by adjusting machine parameters relating to the abnormal region.

12. The method of producing a product according to any one of Claims 1 to 11, comprising the steps of:
comparing product data of a normal product with product data of the product prepared in the production steps, to detect a product, as an abnormal product, having data different from the product data of the normal product;
extracting cause data causing any abnormality from the product data of the abnormal product; and
tracing production pattern data of the equipment data associated with the product data based on the cause data.

13. The method of producing a product according to any one of Claims 1 to 12,
wherein the product is an absorbent article, and
wherein production data of the absorbent article is recorded in association with the absorbent article in the product data stored in the data collection unit.

14. The method of producing a product according to any one of Claims 1 to 12, wherein the statistical value is calculated in each step of a data collection step of the normal product and a data collection step of the abnormal product.

15. The method of producing a product according to any one of Claims 1 to 14, comprising a step of confirming a correlation between image processing data relating to an image processing inspection instrument in the product data, and the equipment data.

16. The method of producing a product according to Claim 15, wherein in the step of confirming a correlation, the correlation is confirmed in in-line.

17. The method of producing a product according to Claim 15, wherein in the step of confirming a correlation, the correlation is confirmed in off-line.

18. The method of producing a product according to any one of Claims 1 to 17, wherein the data collection unit has a main central processing unit and an auxiliary central processing unit.

19. The method of producing a product according to any one of Claims 1 to 18, wherein a set value is registered in advance in the production equipment, in addition to the product data.

20. The method of producing a product according to any one of Claims 1 to 19, wherein the equipment data is configured of:
sheet collection data collected in the period for each product;
long period collection data collected in the period longer than the period of the sheet collection data; and
short period collection data collected in the period shorter than the period of the sheet collection data.

21. The method of producing a product according to any one of Claims 1 to 20, wherein a serial number which identify the product is printed using colored ink or colorless ink.

22. The method of producing a product according to any one of Claims 1 to 21, wherein the product is an absorbent article.

23. The method of producing a product according to any one of Claims 1 to 22, wherein the product is a sheet-form product.

24. An apparatus for producing a product, by which the product is produced correspondingly to a plurality of production steps, comprising:
a plurality of pieces of production equipment by which the product is produced;
a plurality of inspection devices by which the product is inspected;
a sensor for detecting a production pattern possessed by the production equipment; and
a data collection unit in which product data obtained by the inspection devices and production pattern data detected by the sensor are stored in association with each other,
wherein the data collection unit has:
an auxiliary central processing unit in which the product data is processed,
a main central processing unit in which mutually associated equipment data indicating equipment states of the production equipment, auxiliary unit collection data processed in the auxiliary central processing unit and the production pattern data are processed, and
a database server in which main unit collection data processed in the main central processing unit are stored.

25. The apparatus for producing a product according to Claim 24, wherein the auxiliary central processing unit transforms a data format of the product data acquired in the inspection device so as to be able to handle in the main central processing unit.
